# EUROPEAN PATENT APPLICATION

(11) **EP 3 560 418 A1**
(43) Date of publication of application: **30.10.2019**
(21) Application number: 19171280.1
(22) Date of filing: 26.04.2019
(51) Int. Cl.: A61B 5/01, A41D 13/005

(54) **SMART GARMENT**

(30) Priority: 27.04.2018 TW 107114592
(71) Applicant: Intelligence Textile Technology Co., Ltd., 10595 Taipei City (TW)
(72) Inventor: LIN, Chen-Hsiang, 10595 Taipei City (TW)
(74) Representative: DTS Patent- und Rechtsanwälte Schnekenbühl und Partner mbB

(57) **Abstract**

A smart garment (100, 200, 300, 400) has a smart garment body (110), a control module (120) and a sensing module (130). The control module (120) and the sensing module (130) are disposed at the smart garment body (110). The control module (120) electrically connects to the smart garment body (110) for controlling the smart garment body (110). The sensing module (130) has two sensors (131, 132) for obtaining two different sensed data from a wearer and the external environment, respectively. The two different sensed data (DATA1, DATA2) obtained from the wearer and the external environment serve as the basis for controlling the smart garment (100, 200, 300, 400) or are merely used for data analysis.

## Description

### TECHNICAL FIELD

The present disclosure relates to smart garments, and more particularly to a smart garment capable of obtaining two different sensed data from a wearer and external environment, respectively.

### RELATED ART

With the progress of science and technology, many manufacturers are now trying to add electronic components to garments (such as T-shirts, pants, jackets, coats, hats, scarves or shoes) to form smart garments. However, the smart garments have shortcomings. For example, the existing smart garments only sense the data of external environment. Thus, the heating control of the smart garments is imprecise.

### SUMMARY

According to at least one embodiment of the present disclosure, the present disclosure provides a smart garment that can obtain two different sensed data from a wearer and external environment, respectively. Therefore, the two different sensed data can be collected and used for comparing and analyzing or performing a control operation based on the comparing result or analyzing result. For example, in some of the embodiments of the present disclosure, the smart garment controls the temperature thereof more precisely, so as for the smart garment to be more comfortable to the wearer.

According to an embodiment of the present disclosure, a smart garment to be worn by a wearer comprises a smart garment body, a control module disposed at the smart garment body and electrically connected to the smart garment body for controlling the smart garment body and a sensing module disposed at the smart garment body. The sensing module comprises a first sensor for obtaining a first sensed data from the wearer and a second sensor for obtaining a second data from the smart garment, with the first sensed data and the second sensed data serving as a basis for controlling the smart garment body or being merely used for data analysis.

Optionally, the smart garment further has a calculation module disposed at the smart garment body and electrically connected to the sensing module. The calculation module receives the first sensed data and the second sensed data for generating a calculation result. The control module generates a control signal according to the calculation result for controlling the smart garment body automatically.

Optionally, the smart garment further has a communication module disposed at the smart garment body. The communication module enables the smart garment to communicate with an external computer device for transmitting the first sensed data and the second sensed data to the external computer device.

Optionally, the sensing module of the smart garment communicates with the calculation module of the external computer device through the communication module. The calculation module generates the calculation result according to the first sensed data and the second sensed data. The control module generates the control signal according to the calculation result for controlling the smart garment body automatically.

Optionally, the external computer device generates an operation instruction according to an input operation performed by a user, and the control module generates the control signal according to the operation instruction for controlling the smart garment body.

Optionally, the smart garment body has a first signaling yarn having a first staple fiber, a first sheet conductor and an insulating layer. A stretching resistance of the first staple fiber is 26 to 40 strands and the first staple fiber functions as a supporting material. The first sheet conductor is enlacing a surrounding surface of the first staple fiber in a spiral extending manner. An aspect ratio of a cross section of the first sheet conductor corresponding to the spiral extending manner is about 10 to 30, and preferably about 20. The insulating layer surrounds the surrounding surface of the first staple fiber for covering the first sheet conductor and the first staple fiber.

Optionally, a material of the first sheet conductor is one selected from copper-nickel alloy, copper-tin alloy, copper-nickel-silicon alloy, copper-nickel-zinc alloy, copper-nickel-tin alloy, copper-chromium alloy, copper-silver alloy, nickel-brass alloy, phosphor bronze alloy, beryllium copper alloy, nickel-chromium alloy, copper-tungsten alloy and stainless steel.

Optionally, a material of the insulating layer is one selected from polytetrafluoroethylene (PTFE, also known as Teflon®), ethylene tetrafluoroethylene (ETFE), polyethylene terephthalate (PET), polyvinyl chloride (PVC) and polyethylene (PE).

Optionally, a conductive wire is rolled for providing the first sheet conductor, wherein a diameter of a circular section of a first conductive wire is X, a length of the cross section of the first sheet conductor is about 4X, and a width of the cross section of the first sheet conductor is about X/5.

Optionally, the smart garment body has a touch control textile disposed at the smart garment body and electrically connected to the control module. The touch control textile has a plurality of yarns and a plurality of second signaling yarns, and the touch control textile is worn by the yarns and the second signaling yarns. The second signaling yarn has a second staple fiber and a second sheet conductor. A stretching resistance of the second staple fiber is 26 to 40 strands and the second staple fiber functions as a supporting material. The second sheet conductor is enlacing a surrounding surface of the second staple fiber in a spiral extending manner. An aspect ratio of a cross section of the second sheet conductor corresponding to the spiral extending manner is about 10 to 30, and preferably about 20.

Optionally, a material of the second sheet conductor is one selected from copper-nickel alloy, copper-tin alloy, copper-nickel-silicon alloy, copper-nickel-zinc alloy, copper-nickel-tin alloy, copper-chromium alloy, copper-silver alloy, nickel-brass alloy, phosphor bronze alloy, beryllium copper alloy, nickel-chromium alloy, copper-tungsten alloy and stainless steel.

Optionally, a second conductive wire is rolled for providing the second sheet conductor, wherein a diameter of a circular section of the second conductive wire is X, a length of the cross section of the second sheet conductor is about 4X, and a width of the cross section of the second sheet conductor is about X/5.

Optionally, the calculation module uses an artificial intelligence algorithm to generate the calculation result according to the first sensed data and the second sensed data.

In summary, the embodiments of the present disclosure provide a smart garment that is capable of obtaining two different sensed data from the wearer and external environment, respectively.

For better understanding of the features and technical contents of the present disclosure, please refer to the detailed descriptions and drawings of the present disclosure.

### BRIEF DESCRIPTION OF THE DRAWINGS

In order to describe embodiments of the present disclosure or the related art more clearly, drawings are presented. The drawings are only illustrative of the embodiments of the present disclosure, and persons of ordinary skill in the art may still derive other drawings from these drawings without creative efforts.
FIG. 1 is a function block diagram of a smart garment according to an embodiment of the present disclosure;
FIG. 2 is a stereoscopic schematic of the smart garment according to an embodiment of the present disclosure;
FIG. 3 is a function block diagram of a smart garment according to another embodiment of the present disclosure;
FIG. 4 is a function block diagram of a smart garment according to another embodiment of the present disclosure;
FIG. 5 is a function block diagram of a smart garment according to another embodiment of the present disclosure;
FIG. 6 is a schematic diagram of the first cable device for a smart garment according to an embodiment of the present disclosure;
FIG. 7 is a schematic diagram of the second cable device for the smart garment according to an embodiment of the present disclosure;
FIG. 8 is a schematic diagram of the third cable device for the smart garment according to an embodiment of the present disclosure;
FIG. 9 is a schematic diagram of the fourth cable device for the smart garment according to an embodiment of the present disclosure;
FIG. 10 is a configuration schematic diagram of an elastic textile of the smart garment according to an embodiment of the present disclosure;
FIG. 11 is a schematic diagram showing the elastic textile of FIG. 10 is not stretched according to an embodiment of the present disclosure;
FIG. 12 is a schematic diagram showing the elastic textile of FIG. 10 is stretched according to an embodiment of the present disclosure;
FIG. 13 is a configuration schematic diagram of the elastic textile of the smart garment according to another embodiment of the present disclosure;
FIG. 14 is a plan view of a touch control textile according to an embodiment of the present disclosure;
FIG. 15 is a schematic diagram of a box according to an embodiment of the present disclosure;
FIG. 16 is a schematic diagram of an electronic component accommodated in the box according to an embodiment of the present disclosure;
FIG. 17 is a schematic diagram of a front side of a cover according to an embodiment of the present disclosure;
FIG. 18 is a schematic diagram of a back side of the cover according to an embodiment of the present disclosure;
FIG. 19 is a schematic diagram illustrating the cover covering the box according to an embodiment of the present disclosure;
FIG. 20 is a three-dimensional schematic diagram of a signaling yarn according to an embodiment of the present disclosure;
FIG. 21 is a three-dimensional schematic diagram of a signaling yarn according to another embodiment of the present disclosure;
FIG. 22 is a sectional diagram of a signaling yarn according to another embodiment of the present disclosure;
FIG. 23 is diagram illustrating an implementation method of a sheet conductor according to an embodiment of the present disclosure; and
FIG. 24 is a flow chart illustrating a manufacturing method of the signaling yarn according to an embodiment of the present disclosure.

### DETAILED DESCRIPTION OF THE PREFERRED EMBODIMENTS

To make it easier for the examiner to understand the objects, characteristics and effects of this present disclosure, embodiments together with the attached drawings for the detailed description of the present disclosure are provided.

According to an embodiment of the present disclosure, a smart garment is provided. The smart garment is capable of obtaining two different sensed data from a wearer and the external environment, respectively. Therefore, the two different sensed data can be collected and used, such as comparing and analyzing the two different sensed data or performing a control operation based on the comparing result or analyzing result. For example, a temperature sensed data of the wearer (apparent temperature) and a temperature sensed data of the external environment (ambient temperature) can be collected, that a temperature of a smart garment body can be controlled by comparing and analyzing the temperature sensed data of the wearer and the temperature sensed data of the external environment.

To make it easier for the examiner to understand the objects, characteristics and effects of this present disclosure, embodiments together with the accompanying drawings for the detailed description of the present disclosure are provided.

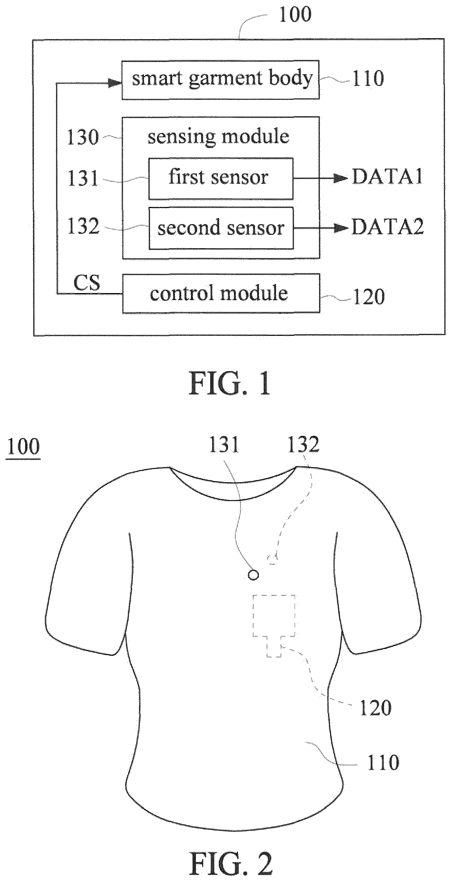

Please refer to FIG.1 and FIG. 2, FIG. 1 is a function block diagram of a smart garment according to an embodiment of the present disclosure, and FIG. 2 is a stereoscopic schematic of the smart garment according to an embodiment of the present disclosure. As shown in FIG. 1 and FIG. 2, the smart garment 100 has a smart garment body 110, a control module 120 and a sensing module 130. The smart garment body 110 is to be worn by a wearer. The control module 120 is disposed at the smart garment body 110. The control module 120 is electrically connected to the smart garment body 110 for controlling the smart garment body 110. The sensing module 130 is disposed at the smart garment body 110. The sensing module 130 has a first sensor 131 and a second sensor 132. The first sensor 131 obtains a first sensed data DATA1 from the wearer. The second sensor 132 obtains a second sensed data DATA2 from the external environment of the smart garment 100. Therefore, the first sensed data DATA1 and the second sensed data DATA2 are collected and used by the first sensor 131 and the second sensor 132, such as comparing the first sensed data DATA1 to the second sensed data DATA2, analyzing the first sensed data DATA1 and the second sensed data DATA2 or performing a control operation based on the comparing result or analyzing result.

In the embodiment, the smart garment body 110 is a kid garment, but is not intended to limit the present disclosure. The smart garment body 110 can be pet clothes or garments (T-shirts, pants, jackets, coats, hats, scarves, skirts, shoes or socks) for children or adults.

In the embodiment, the control module 120 is electrically connected to the smart garment body 110 for providing the power to the smart garment body 110 and controlling the smart garment body 110. For example, the control module 120 controls the temperature of the smart garment body 110. The control module 120 may have a controller (e.g., microcontroller).

In the present embodiment, the first sensor 131 and the second sensor 132 are disposed approximately on the inner (inside) and outer (outside) surfaces of the chest position of the smart clothing body 110. However, the present disclosure is not intended to limit the position of the first sensor 131 and the second sensor 132. Moreover, the present disclosure also is not intended to limit the numbers of the sensors of the sensing module 130 and the sensing module 130 may have two or more sensors.

In the embodiment, the first sensor 131 collects the temperature sensed data of the wearer (apparent temperature), and the second sensor 132 collects the temperature sensed data of the external environment (ambient temperature). However, the present disclosure is not to limit the type of the first sensor 131 and the second sensor 132. For example, the first sensor 131 and the second sensor 132 collect a humidity sensed data of the wearer (apparent humidity) and a humidity sensed data of the external environment (ambient humidity) respectively. For another example, the first sensor 131 collects a heart rate sensed data or a respiratory rate sensed data of the wearer, and the second sensor 132 collects the temperature sensed data and the humidity sensed data of the external environment.

Compared with existing smart garments, the smart garment of the present disclosure is capable of collecting the sensed data from the wearer and the external environment simultaneously. The sensed data collected by the smart garment of the present disclosure can be applied to the big data analytics to improve the production process of the smart garment, adjust the material or position of components in the smart garment, correct the control mode or parameters of the control module and analyze the impact of different environments for the smart garments (the control elements, the control mode or the control parameters of the smart garment in tropical and frigid climates may need to be adjusted).

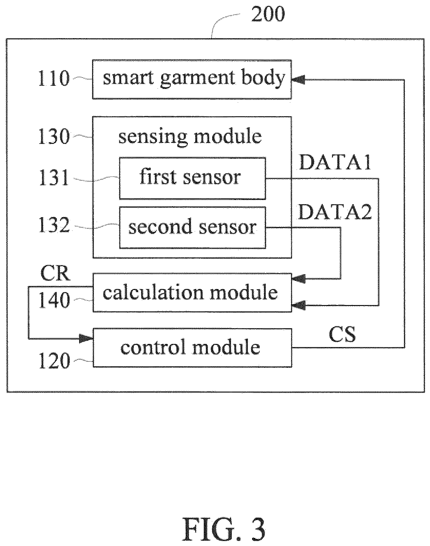

Please refer to FIG. 3, and FIG. 3 is a function block diagram of a smart garment according to another embodiment of the present disclosure. The elements of the smart garment 200 are substantially same as the elements of the smart garment 100. The smart garment 200 further has a calculation module 140 disposed at the smart garment body 110 and electrically connected to the sensing module 130. The calculation module 140 receives the first sensed data DATA1 and the second sensed data DATA2 for generating a calculation result CR according to the first sensed data DATA1 and the second sensed data DATA2. The control module 120 generates a control signal CS according to the calculation result CR received from the calculation module 140 for controlling the smart garment body 110 automatically. Since the control module 120 of the embodiment controls (such as increasing temperature, decreasing temperature, increasing humidity or reducing humidity) the smart garment body 110 according to the calculation result CR that is from an internal component, the control mentioned above can be completed without assistance of external components or manual operation of a user.

In the embodiment, the calculation module 140 and the control module 120 can be implemented as a single element (e.g., a single microcontroller), but is not intended to limit the present disclosure. In another embodiment, the calculation module 140 and the control module 120 are implemented as different elements and are capable of communicating with each other in multiple ways.

Optionally, in the embodiment, the calculation module 140 uses an artificial intelligence algorithm (for example, building computational models through neural network technology) to generate the calculation result CR according to the first sensed data DATA1 and the second sensed data DATA2, but is not intended to limit the present disclosure. For example, the calculation module 140 is capable of generating the calculation result CR by Fuzzy Control or other method.

Compared with existing smart garments, the smart garment of the present disclosure is advantageous in that the temperature is controlled accurately by the calculation module 140 that integrating and analyzing the sensed data of the wearer and the external environment and transmitting the calculation result CR to the control module 120.

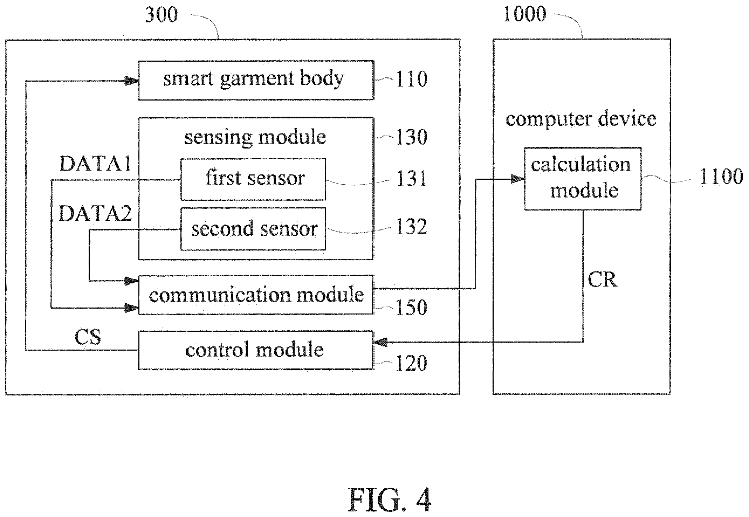

Please refer to FIG. 4, and FIG. 4 is a function block diagram of a smart garment according to another embodiment of the present disclosure. The elements of a smart garment 300 are substantially same as the elements of the smart garment 100. The smart garment 300 further has a communication module 150 disposed at the smart garment body 110. The communication module 150 enables the smart garment 300 to communicate with an external computer device 1000 for transmitting the first sensed data DATA1 and the second sensed data DATA2 to the computer device 1000. Thus, the first sensed data DATA1 and the second sensed data DATA2 can be transmitted to outside of the smart garment body 110 for collecting and using (such as comparing and analyzing the two different sensed data or performing a control operation based on the comparing result or analyzing result) the two different sensed data through the communication module 150.

Optionally, in the embodiment, the computer device 1000 has a calculation module 1100. The sensing module 130 of the smart garment 300 communicates with the calculation module 1100 of the computer device 1000 disposed at outside through the communication module 150. The calculation module 1100 generates the calculation result CR according to the first sensed data DATA1 and the second sensed data DATA2. The calculation result CR of the calculation module 1100 is transmitted back to the control module 120 of the smart garment 300 through the communication module 150. The control module 120 generates the control signal CS according to the calculation result CR for controlling the smart garment body 110 automatically. Since the control module 120 of the embodiment controls the smart garment body 110 according to the calculation result CR from external components, the control (for example, increase temperature, decrease temperature, increase humidity or reduce humidity of the smart garment body 110) mentioned above can complete automatically with no manual operation from the user.

Optionally, in the embodiment, the calculation module 1100 uses an artificial intelligence algorithm (for example, building computational models through neural network technology) to generate the calculation result CR according to the first sensed data DATA1 and the second sensed data DATA2, but is not intended to limit the present disclosure. For example, the calculation module 1100 can generate the calculation result CR by Fuzzy Control or other method.

Compared with existing smart garments, the smart garment of the present disclosure is advantageous in that the temperature is controlled accurately by the calculation module 1100 that integrates and analyzes the sensed data of the wearer and the external environment for obtaining the calculation result CR and transmits the calculation result CR to the control module 120.

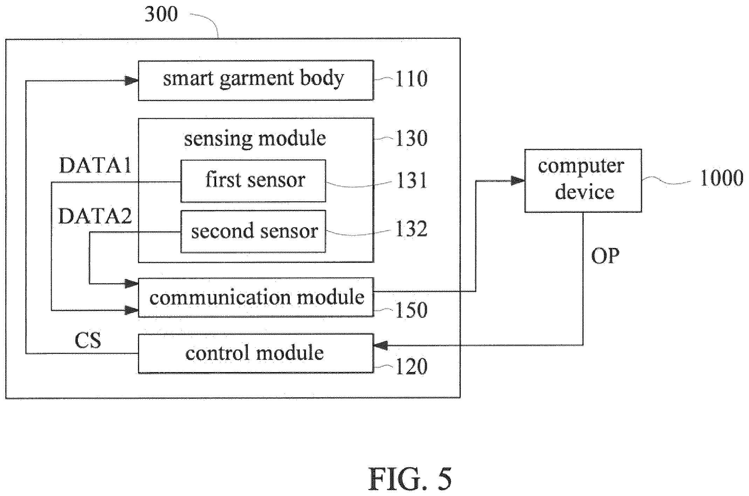

Please refer to FIG. 5, and FIG. 5 is a function block diagram of a smart garment according to another embodiment of the present disclosure. The elements of a smart garment 400 are substantially same as the elements of the smart garment 300. The smart garment 400 may or may not have the calculation module. The computer device 1000 generates an operation instruction OP according to an input operation performed by the user. The control module 120 generates the control signal CS according to the operation instruction OP for controlling the smart garment 110, such as, increase temperature, decrease temperature, increase humidity or reduce humidity. The control mentioned above can be completed automatically through the manual operation from the user. In addition, in the embodiment which the smart garment 400 has the calculation module, the operation instruction OP generated according to the input operation performed by the user can function as one of a raw data of the artificial intelligence algorithm. For example, the calculation module modifies the computational operational model according to the operation instruction OP.

In the embodiment, the computer device 1000 is a mobile phone. The mobile phone installed with an application. The application enables a user (not limited to the wearer) to perform the input operation through a screen of the mobile phone and thus generate the operation instruction OP. However, the present disclosure is not limited thereto. The computer device 1000 may be a tablet, personal computer or other computer device that enables the user to perform the input operation.

A point worth noting is that the automatic control related to the control signal CS generated according to the calculation result CR and the automatic control related to the control signal CS generated according to the operation instruction OP are not mutually exclusive. In other words, in some embodiments, the control module of the smart garment can have the automatic control and the manual control at the same time. For example, when the smart garment enables the control module to perform heating control according to the calculation result CR, the user performs the input operation to further adjust the intended temperature and speed of the heating process.

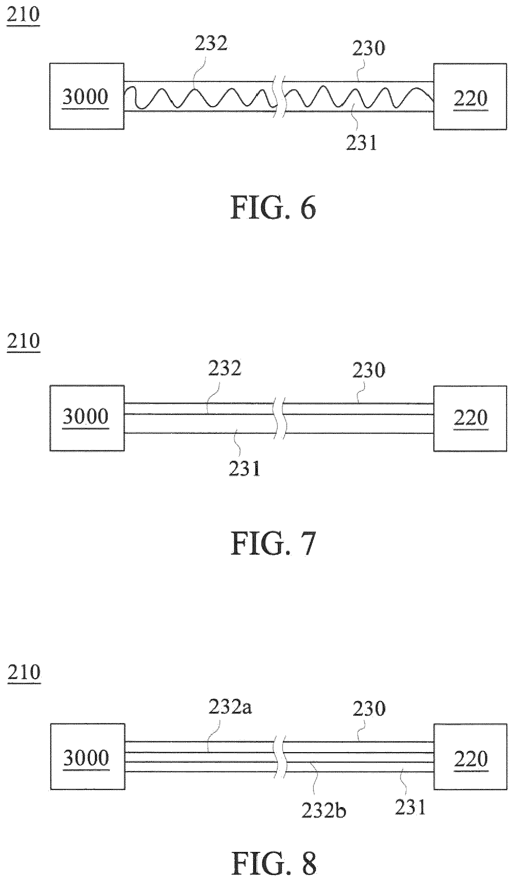

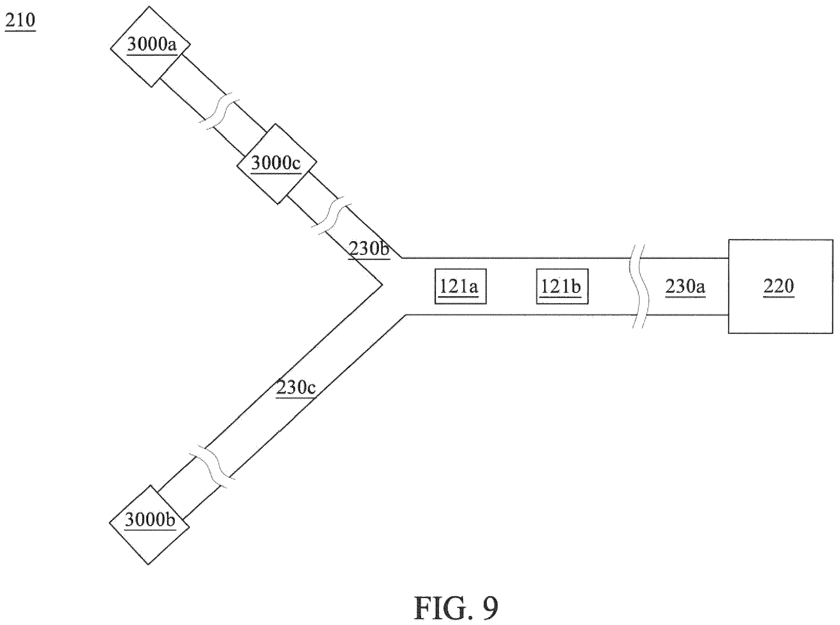

The embodiments mentioned above and other embodiments of the smart garment may have a cable device 210 for connecting to one or more external electrical components 3000. For example, two or more electrical components 3000 are disposed at two or more ends of the cable device 210, respectively. The cable device 210 can be sewn to the smart garment after being made individually or made with the smart garment and become a part of the smart garment. Referring to FIGS. 6-9, FIG. 6 is a schematic diagram of the first cable device for a smart garment according to an embodiment of the present disclosure. FIG. 7 is a schematic diagram of the secondcable device for the smart garment according to an embodiment of the present disclosure. FIG. 8 is a schematic diagram of the third cable device for the smart garment according to an embodiment of the present disclosure. FIG. 9 is a schematic diagram of the fourth cable device for the smart garment according to an embodiment of the present disclosure.

The cable device 210 has a first connector 220 and a connecting cable 230, and the first connector 220 is electrically connected to one end of the connecting cable 230 and the electrical component 3000 is electrically connected to the other end of the connecting cable 230. Electric signals are propagated between the electrical component 3000 and the first connector 220 through the connecting cable 230. Although the cable device 210 in the embodiment has the connector (first connector 220), but is not intended to limit the present disclosure. For example, in other embodiment, the cable device 210 may have no connector.

In the embodiment, the first connector 220 is a first connector plug for being electrically connected to the electrically device relating to the first connector plug. The first connector 220 is one selected from USB Type-A plug, USB Type-C plug, USB Micro-B plug, USB Mini-B plug, magnetic plug, Lightning plug and TRS connector, but the present disclosure is not limited thereto. The electronic device is, for example, a portable electronic device, a computer host, a power bank, and the like, but the present disclosure is not limited thereto.

The connecting cable 230 has a first textile 231 and a conductive wire element 232. The first textile 231 is elastic material or non-elastic material. The conductive wire element 232 is disposed within the first textile 231 periodically. One end of the conductive wire element 232 is electrically connected to the first connector 220, and the other end of the conductive wire element 232 is electrically connected to the electrical component 3000. Therefore, the electric signals or electrical energy can be propagated between the first connector 220 and the electrical component 3000 through the conductive wire element 232. The first textile 231 is disposed between the first connector 220 and the electrical component 3000 for connecting the first connector 220 to the electrical component 3000, and the conductive wire element 232 is disposed within the first textile 231 to be electrically connected to the first connector 220 and the electrical component 3000.

In the embodiment illustrated by FIG. 6, the first textile 231 is implemented as the elastic material, and the conductive wire element 232 is periodically disposed within the first textile 231 in a wavy manner (for example, sine curve manner). In the embodiment, a stretching space is provided by the conductive wire element 232 disposed within the first textile 231 in the wavy manner. Thus, when the first textile 231 is stretched under an external force, the conductive wire element 232 can be stretched together with the first textile 231 without breaking under the external force. In another embodiment illustrated by FIG. 7, the first textile 231 is made of the non-elastic material, and the conductive wire element 232 is periodically disposed within the first textile 231 in a straight-line manner.

In the embodiment illustrated by FIG. 8, the connecting cable 230 further has multiple conductive wire elements 232 according to different requirements. The connecting cable 230 in the embodiment has a conductive wire element 232a and a conductive wire element 232b. However, the present disclosure is not limited by the number of the conductive wire element 232 of FIG. 6, FIG. 7 or FIG. 8.

In the embodiment illustrated by FIG. 9, the connecting cable 230 further has a first sub connecting cable 230a, a second sub connecting cable 230b and a third sub connecting cable 230b. One end of the first sub connecting cable 230a is electrically connected to the first connector 220, and the other end of the first sub connecting cable 230a is electrically connected to one end of the second sub connecting cable 230b and one end of the third sub connecting cable 230c. The first sub connecting cable 230a is controlled by at least one control element 121 of the control module 120. In the embodiment, the first sub connecting cable 230a has a control element 121a and 121b, but the present disclosure is not limited thereto. In the embodiment, the connecting cable 230 has multiple electrical components 3000. For example, the other end of the second sub connecting cable 230b is electrically connected to an electrical component 3000a and an electrical component 3000c, the other end of the third sub connecting cable 230c is electrically connected to an electrical component 3000b, and the present disclosure is not limited thereto. In other words, the cable device 210 can have multiple connecting cables 230 and the control element 121 according to the requirements for increasing functional features of the cable device 210, but the present disclosure is not limited to the embodiment illustrated by FIG. 9.

In the embodiment, the conductive wire element 232 is made of the signaling yarn or enameled wire, but the present disclosure is not limited thereto. Moreover, the conductive wire element 232 of a variable type can be disposed within the connecting cable 230. For example, in the embodiment illustrated by FIG. 8, the conductive wire element 232a can be implemented by the signaling yarn, whereas the conductive wire element 232b can be implemented by the enameled wire, but the present disclosure is not limited thereto.

In the embodiment, the enameled wire has an insulating paint, a material of the insulating paint is one selected from polytetrafluoroethylene (PTFE, i.e. Teflon®), ethylene tetrafluoroethylene (ETFE), polyethylene terephthalate (PET), polyvinyl chloride (PVC), polyethylene (PE) and other polymer insulating materials, but the present disclosure is not limited thereto.

In the embodiment, the first textile 231 is one selected from polyester, polyamide, polyacrylonitrile, polyethylene, polypropylene, cellulose, protein, elastic fiber, poly perfluoroethylene, polyparaphenylene benzoxazole, polyether ketone, carbon and glass fiber, but the present disclosure is not limited thereto.

In the embodiment, the electrical component 3000 can be implemented as a speaker or audio signal receiving element. For example, the electrical component 3000 can be the speaker of headphones or the audio signal receiving element of microphone.

In the embodiment, the electrical component 3000 can be a converter. For example, the electrical component 3000 is one selected from card reader, RJ45 converter, 30 pin converter, TRS converter, HDMI converter, VGA converter and USB converter, but the present disclosure is not limited thereto.

In the embodiment, the electrical component 3000 can be a second connector plug. For example, the electrical component 3000 is one selected from USB Type-A plug, USB Type-C plug, USB Micro-B plug, USB Mini-B plug, magnetic plug and Lightning plug, but the present disclosure is not limited thereto.

In the embodiment, the electrical component 3000 can be a battery device (for example, power bank), and the present disclosure is not limited thereto.

In the embodiment, the electrical component 3000 is of any appropriate type selected as needed and in accordance with the first connector 220. For example, when the first connector 220 is USB Type-C plug, the electrical component 3000 is one selected from card reader, USB Type-A plug and USB converter accordingly, but the present disclosure is not limited thereto.

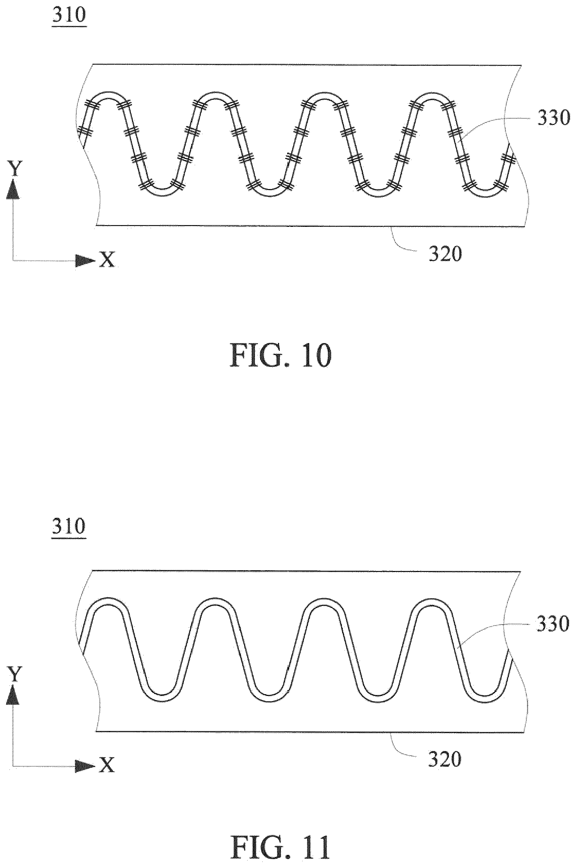

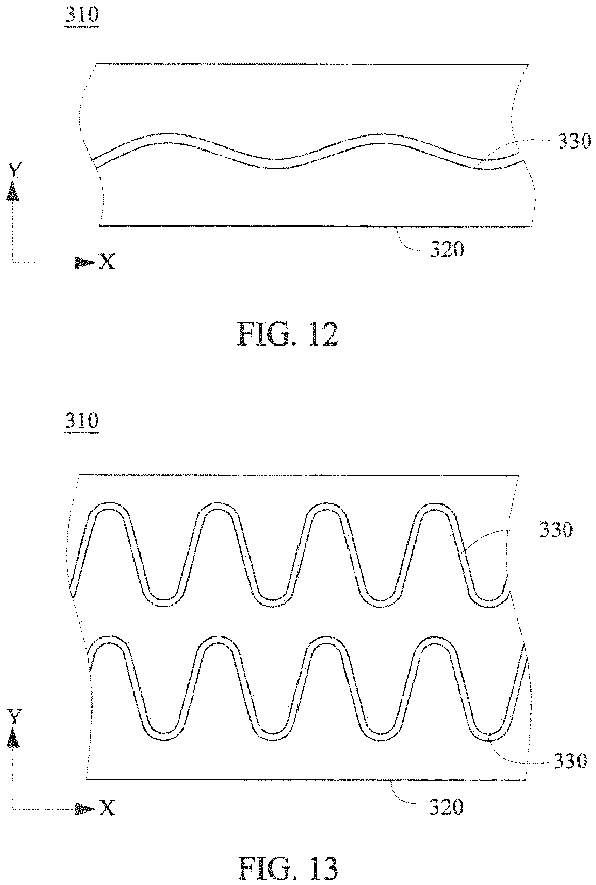

When the first textile 231 is the elastic material, the first textile and the conductive wire element can be woven into an elastic textile. Please refer to FIGS. 10-13. FIG. 10 is a configuration schematic diagram of an elastic textile of the smart garment according to an embodiment of the present disclosure. FIG. 11 is a schematic diagram showing the elastic textile of FIG. 10 is not stretched according to an embodiment of the present disclosure. FIG. 12 is a schematic diagram showing the elastic textile of FIG. 10 is stretched according to an embodiment of the present disclosure. FIG. 13 is a configuration schematic diagram of the elastic textile of the smart garment according to another embodiment of the present disclosure.

As shown in FIG. 10, the elastic textile 310 comprises an elastic webbing 320 and at least one conductive wire element 330. The elastic webbing 320 has at least one layer. In an embodiment, the elastic webbing 320 is implemented by spandex fiber, but is not intended to limit the present disclosure. In an embodiment, the elastic webbing 320 is not limited to have one layer. For example, the elastic webbing 320 can have double or multiple layers. The at least one conductive wire element 330 is fixed in place within the elastic webbing 320 in a wavy manner (for example, a sinusoidal curve manner) for providing a stretching space. Because of the stretching space, the at least one conductive wire element 330 is unlikely to sever even if the elastic webbing 320 is stretched under external forces.

As shown in FIG. 11, before the elastic textile 310 is stretched, the at least one conductive wire element 330 is presented on the elastic webbing 320 in the wavy manner. As shown in FIG. 12, when the elastic textile 310 is stretched under external forces, the at least one conductive wire element 330 is stretched in first direction X as the elastic webbing 320 is stretched under the external forces. Distances between multiple peaks formed by the at least one conductive wire element 330 disposed on the elastic webbing 320 in the wavy manner increase under the external forces. Thus, the at least one conductive wire element 330 can be stretched together with the elastic webbing 320 without breaking under the external pulling forces.

As shown in FIG. 13, the elastic textile 340 according to another embodiment of the present disclosure has two conductive wire elements 330 (330a and 330b) disposed on the elastic webbing 320, the conductive wire element 330a can be implemented by enameled wire, and the conductive wire element 330b can be implemented by signaling yarn. In other words, the elastic textile 340 comprises different numbers and/or types of conductive wire element 330 disposed on same elastic webbing 320 according to different requirements, and the numbers and types of conductive wire element 330 of FIG. 9, FIG. 10, FIG. 11 or FIG. 12 are not intended to limit the present disclosure.

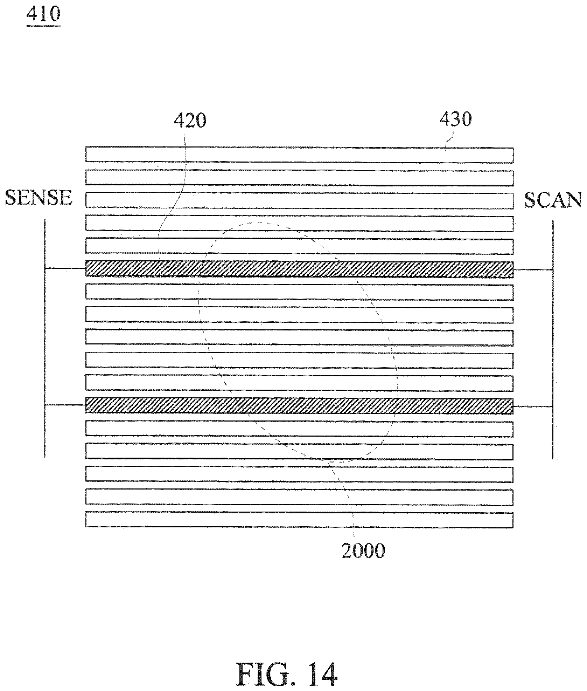

The smart garment of the embodiments mentioned above and other embodiments may have a touch control textile 410. Please refer to FIG. 14, and FIG. 14 is a plan view of a touch control textile according to an embodiment of the present disclosure.

The touch control textile 410 can be formed by weaving a plurality of signaling yarns 420 and a plurality of yarns 430, wherein the outer surface of each signaling yarn 420 has no insulating layer. Every two adjacent signaling yarns 420 are spaced apart by five yarns 430. The signaling yarns 420 and the yarns 430 are extending in a horizontal direction and arranged in sequence in the vertical direction. However, the aforesaid spacing and arrangement of the signaling yarns 420 and the yarns 430 are not restrictive of the present disclosure and thus are subject to changes as needed.

One end of the signaling yarn 420 receives a scan signal SCAN transmitted from the control module 120, and the control module 120 receives a touch control signal SENSE transmitted from another end of the signaling yarn 420, and the control module 120 determines whether the touch object 2000 (such as, a finger or other touch object) contacts the touch control textile 410 according to the touch control signal SENSE. Since the signaling yarn 420 has no insulating layers covering it outer surface, when the touch object 2000 contacts the touch control textile 410, the induced resistance changes the touch control signal SENSE, such that the control module 120 can determine whether the touch object 2000 contacts the touch control textile 410 according to the touch control signal SENSE. That is, the signaling yarn 420 is provided as the resistance touch control sensing device.

In addition, a material of the yarn 430 can be selected from one of a polyester, a polyamide, a polyacrylic, a polyethylene, a polypropylene, a cellulose, a protein, an elastomeric, a polytetrafluoroethylene, a poly-p-phenylenebenzobisthiazole (PBO), a polyetherketone, a carbon and a glass fiber, but the present disclosure is not limited thereto.

In the embodiment, the touch control textile 410 functions as a control switch. For example, the touch control textile 410 controls the temperature, by functioning as a switch for disabling or enabling a temperature control function or a switch for increasing or decreasing the temperature. In brief, the controller can determine whether the touch control textile 410 be touched and generate a control signal for controlling the temperature. Please note that, in other embodiment, the smart garment can have no touch control textile 410 but have other control switch to implement the function mention above.

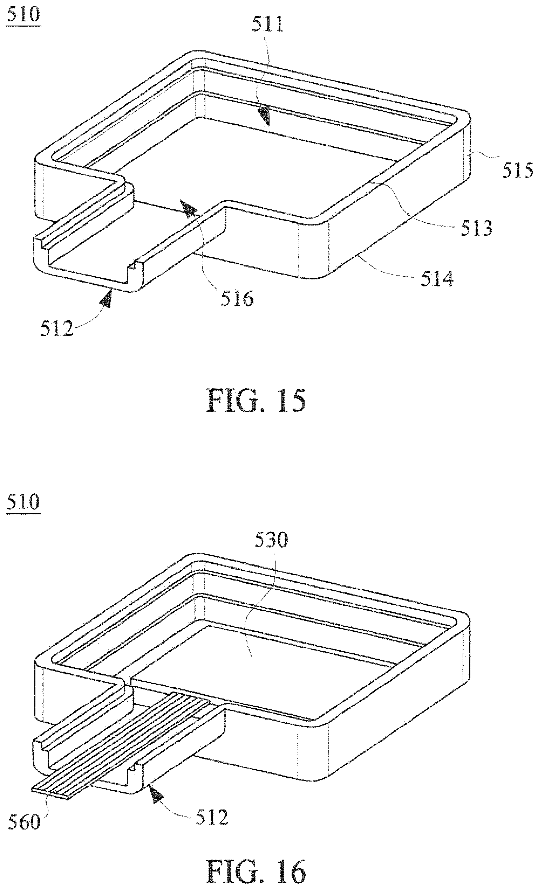

The smart garment of the embodiment mentioned above and other embodiments may have an electrical component protection device. An embodiment of the electrical component protection device 500 will be described below with FIGS. 15-19. The electrical component protection device 500 has a box 510 and a cover 520. An electrical component 530 is accommodated in the electrical component protection device 500 to form an electronic component module. The materials for the box 100 and the cover 200 are ones selected from rubber, silicone and other material that is waterproof and resistant to high temperatures. In an embodiment, the electrical component 530 can be the control module 120. In another embodiment, the electrical component 530 can be the combination of the control module 120 and the calculation module.

Referring to FIG. 15, the box 510 has an accommodating groove 511 and only one outlet wire groove 512. The accommodating groove 511 has an opening 513, a bottom 514 and a side wall 515. The side wall 515 is disposed between the opening 513 and the bottom 514 to form the accommodating groove 511. The outlet wire groove 512 is disposed on the side wall 515 of the accommodating groove 511 and closing to one side of the opening 513. The outlet wire groove 512 is coupled to an outlet wire groove opening 516 of the side wall 515 and protrudes from the accommodating groove 511.

As shown in FIG. 16, the electronic component 530 is accommodated in the accommodating groove 511 and electrically connected to one end of at least one conductive wire element 560. When sorted, the at least one conductive wire 560 can be disposed in the outlet wire groove 512 through the outlet wire groove opening 516, and the at least one conductive wire element 560 extends to the outside of the electronic component protection device 500. In an embodiment of the present disclosure, the electronic component 530 is one selected from a chip module, a printed circuit board and a battery element, but the present disclosure is not limited thereto.

Therefore, another end of the at least one conductive wire element 560 is electrically connected to at least one textile conductive wire of the outside of the electronic component protection device 500 through the outlet wire groove 512. The at least one textile conductive wire is conductive fabric, for example, nano-silver wire or conductive yarn, but the type of the textile conductive wire is not intended to limit the present disclosure.

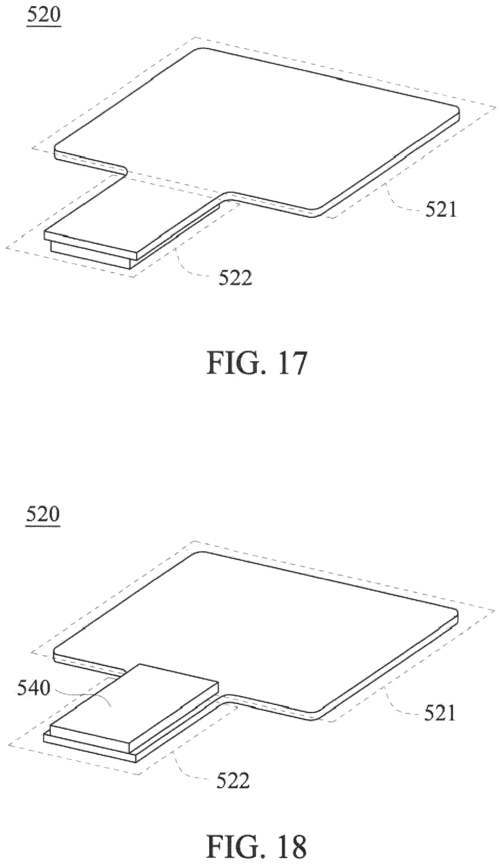

Please refer to FIG. 17 and FIG. 18, FIG. 17 is a schematic diagram of a front side of the cover 520, and FIG. 18 is a schematic diagram of a back side of the cover 520. The cover 520 comprises a first cover portion 521 and a second cover portion 522. The first cover portion 521 covers the opening 513 of the box 510. The second cover portion 522 covers the outlet wire groove 512. The second cover portion 522 is coupled to a part of the first cover portion 521 and protrudes from the first cover portion 521. A protrusion 540 is provided on the back side of the second cover portion 522 of the cover 520.

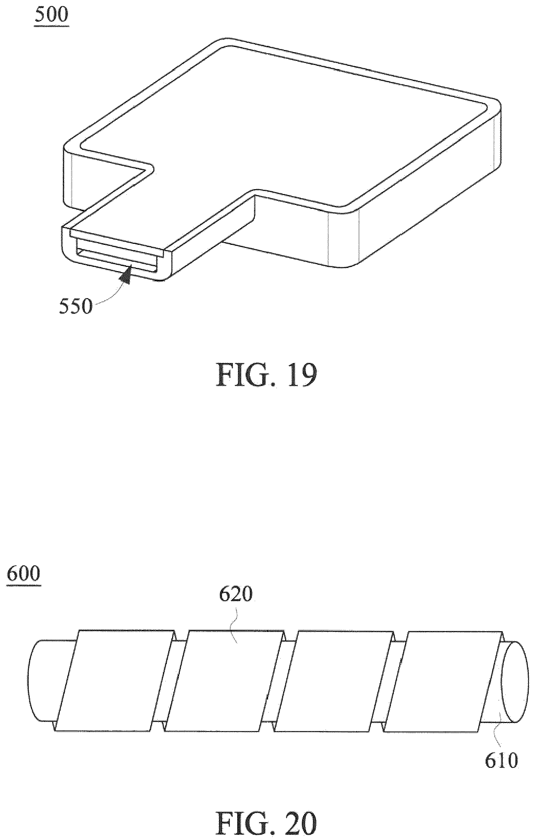

As shown in FIG. 19, when the cover 520 covers the box 510, the back side of the first cover portion 521 faces and covers the opening 513 of the box 510, and the back side of the second cover portion 522 faces and covers the outlet wire groove 512. Simultaneously, the protrusion 540 is coupled to the outlet wire groove 512 to form an outlet wire accommodating space 550. Therefore, the at least one conductive wire element 560 disposed in the outlet wire groove 512 can extend to the outside of the electronic component protection device 500 through the outlet wire accommodating space 550.

In the embodiment, when the electronic component 530 is accommodated in the accommodating groove 511 and the at least one conductive wire element 560 is sorted and accommodated in the outlet wire groove 5 12, the box 520 and cover 510 can be smeared with waterproof glue. Thus, the cover 520 covers and sticks to the box 510 by the waterproof glue, and the waterproof glue fills a vacant space between the outlet wire groove 512 and the conductive wire element 560.

The shape of the accommodating groove 511 and the outlet wire groove 512 are polygonal, round or oval. The cover 520 corresponds in shape to the box 510. As shown in FIG. 19, in the embodiment, the accommodating groove 511 and the outlet wire groove 512 are quadrilateral, whereas the first cover portion 521 and the second cover portion 522 are also quadrilateral so as to correspond in shape to the accommodating groove 511 and the outlet wire groove 512. In an embodiment of the present disclosure, lengths of a first side and a second side (adjacent to the first side) of the accommodating groove 511 are the same, for example, the lengths are 3 cm, and a length of a long side of the outlet wire groove 512 is 1.5 cm and a length of a short side of the outlet wire groove 512 is 1 cm. In this regard, the aforementioned lengths of the first side, the second side, the long side and the short side are merely exemplary, but the present disclosure is not limited thereto.

The embodiments and implementation method of the signaling yarn of the present disclosure will be described below with the following figs.

Please refer to the FIG. 20, and FIG. 20 is a three-dimensional schematic diagram of a signaling yarn of an embodiment of the present disclosure. The signaling yarn 600 comprises a staple fiber 610 and a sheet conductor 620, and the staple fiber 610 is provided as a supporting material to support the sheet conductor 620 enlacing the staple fiber 610. The sheet conductor 620 is enlacing a surrounding surface of the staple fiber 610 in a spiral extending manner to increase a stretching resistance of the signaling yarn 600.

Optionally, the stretching resistance of the signaling yarn 600 can be further increased by selecting the strength of the staple fiber 610 and / or an aspect ratio of a cross section of the sheet conductor 620 corresponding to the spiral extending manner. In this embodiment, the strength of the staple fiber 610 is selected to be 30 strands, and the aspect ratio of the cross section of the sheet conductor 620 corresponding to the spiral extending manner is selected to be about 20, but the present disclosure is not limited thereto. For example, the staple fiber 610 may have the strength of 26, 28, or 40 strands, or the aspect ratio of the cross section of the sheet conductor 620 corresponding to the spiral extending manner may be selected to be about between 10 and 30.

In the embodiment, a material of the staple fiber 610 is one selected from polyester, polyamides, polyacrylonitriles, polyethylenes, polypropylenes, celluloses, proteins, elastic fibers, poly perfluoroethylene, polyparaphenylene benzoxazole, polyether ketone, carbon and glass fiber, but the present disclosure is not limited thereto. The material of the short staple fiber 610 can be selected as needed.

In the embodiment, a material of the sheet conductor 620 is alloy, such as copper-nickel alloy, copper-tin alloy, copper-nickel-silicon alloy, copper-nickel-zinc alloy, copper-nickel-tin alloy, copper-chromium alloy, copper-silver alloy, nickel-brass alloy, phosphor bronze alloy, beryllium copper alloy, nickel-chromium alloy, copper-tungsten alloy, stainless steel and other commercially conductive alloys, but the present disclosure is not limited thereto. In different embodiments, the type of alloy may have different options. For example, the signaling yarn 600 can be used as a touch sensing element in a touch control textile. One end of the signaling yarn 600 receives a scan signal and the other end of the signaling yarn 600 transmits a touch sensing signal. Therefore, a smaller resistance value of the alloy can be selected as the material of the sheet conductor 620.

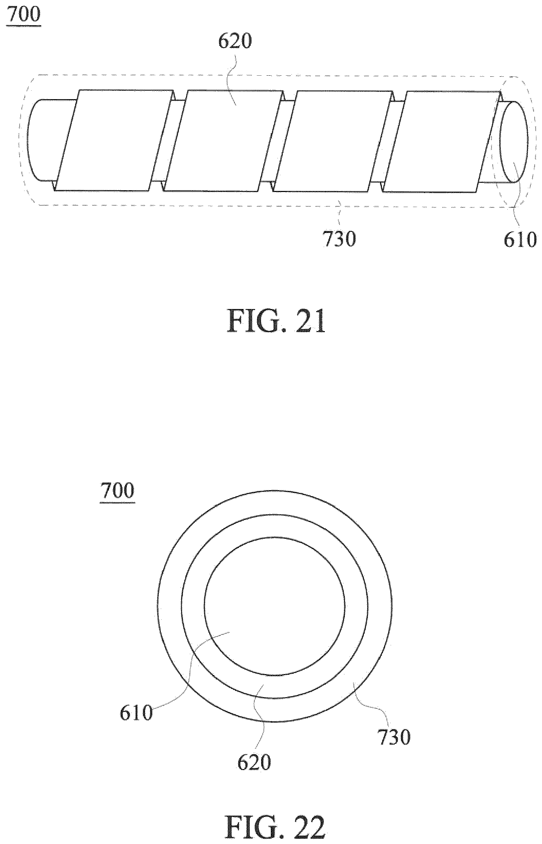

Referring to FIG. 21, FIG. 21 is a three-dimensional schematic diagram of a signaling yarn according to another embodiment of the present disclosure. Compared with the signaling yarn 600 in the embodiment illustrated by FIG. 20, the signaling yarn 700 provided in the embodiment of FIG. 21 further has an insulating layer 730 and the insulating layer 730 surrounds the surrounding surface of the staple fiber 610 so as to cover the sheet conductor 620 and the staple fiber 610. Since the sheet conductor 620 and the staple fiber 610 of the signaling yarn 700 of FIG. 21 are the same as the sheet conductor 620 and the staple fiber 610 of the signaling yarn 600 of FIG. 20, they are not, for the sake of brevity, described herein.

In the embodiment, a material of the insulating layer 730 is one selected from polytetrafluoroethylene, ethylene tetrafluoroethylene, polyethylene terephthalate, polyvinyl chloride, polyethylene and other polymer insulation materials, but the present disclosure is not limited thereto. The material of the sheet conductor 620 and the insulating layer 730 can be selected as needed For example, the signaling yarn 700 can be used as a heating element for heating textiles, so the sheet conductor 620 can be made of an alloy with a large resistance value, and the insulating layer 730 can be made of an insulating material with high heat resistance (for example, polytetrafluoroethylene).

Referring to FIG. 22, FIG. 22 is a sectional diagram of a signaling yarn according to another embodiment of the present disclosure. In the sectional view of the signaling yarn 700, according to the descriptions above, the staple fiber 610 is provided as the support material of a central layer, and the other two layers beside the staple fiber 610 are sequentially the sheet conductor 620 and the insulating layer 730. The signaling yarn 700 of the embodiment has only one sheet conductor 620 and one insulating layer 730, but the present disclosure is not limited thereto. In other embodiments, there may be more layers of sheet conductors and insulating layers, for example, six layers or eight layers, and the number of layers may vary as needed.

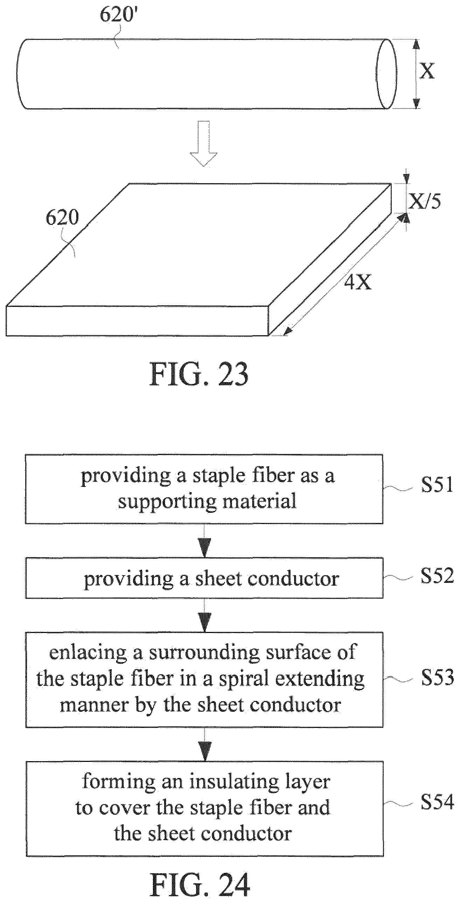

Referring to FIG. 23, FIG. 23 is diagram illustrating an implementation method of a sheet conductor of an embodiment of the present disclosure. In this embodiment, a length and a width of the cross section of the sheet conductor 620 are approximately 4X and X/5 respectively, wherein X is a diameter of the circular cross-section of a conductive wire 620'. The conductive wire 620' is rolled by a rolling mill to form the sheet conductor 620. However, the formation of the sheet conductor 620 is not intended to be a limitation of the present disclosure. In other words, there are different implementations of the sheet conductor 620 of the embodiment of the present disclosure.

Please refer to FIG. 21 and FIG. 24, FIG. 24 is a flow chart illustrating a manufacturing method of the signaling yarn according to an embodiment of the present disclosure. First, in step S51, a staple fiber 610 is provided as a supporting material, wherein the staple fiber 610 has a stretching resistance of 26 to 40 strands. Next, in step S52, a sheet conductor 620 is provided. Next, in step S53, the sheet conductor 620 enlaces a surrounding surface of the staple fiber 610 in the spiral extending manner, wherein an aspect ratio of a cross section of the sheet conductor 620 corresponding to the spiral extending manner is about 10 to 30, and preferably about 20. Finally, in step S54, an insulating layer 730 is formed and surrounds the surrounding surface of the staple fiber 610 to cover the staple fiber 610 and the sheet conductor 620, and thus the signaling yarn 700 of FIG. 21 is manufactured.

Furthermore, in order to manufacture the signaling yarn 600 of FIG. 20 by using the descriptions of the manufacturing method of the signaling yarn of FIG. 24, only steps S51 to S53 of FIG. 24 need to be executed. In addition, the implementation of the sheet conductor 620 provided in step S52 can be the same as the implementation shown in FIG. 23, but the present disclosure is not limited thereto.

In summary, the present disclosure provides a smart garment that can obtain two different sensed data from the wearer and the external environment, respectively.

## Claims

1. A smart garment (100, 200, 300, 400), to be worn by a wearer, comprising:
a smart garment body (110);
a control module (120), disposed at the smart garment body (110), and electrically connected to the smart garment body (110) for controlling the smart garment body (110); and
a sensing module (130), disposed at the smart garment body (110),
wherein the sensing module (130) comprises a first sensor (131) for obtaining a first sensed data (DATA1) from the wearer and a second sensor (132) for obtaining a second sensed data (DATA2) from an external environment of the smart garment (100, 200, 300, 400), with the first sensed data (DATA1) and the second sensed data (DATA2) serving as a basis for controlling the smart garment body (110) or being merely used for data analysis.

2. The smart garment (100, 200, 300, 400) according to claim 1, further comprising:
a calculation module (140, 1100), disposed at the smart garment body (110) and electrically connected to the sensing module (130) for receiving the first sensed data (DATA1) and the second sensed data (DATA2) and generating a calculation result (CR) accordingly, wherein the control module (120) generates a control signal (CS) according to the calculation result (CR) for controlling the smart garment body (110) automatically.

3. The smart garment (100, 200, 300, 400) according to claim 2, wherein the calculation module (140, 1100) uses an artificial intelligence algorithm to generate the calculation result (CR) according to the first sensed data (DATA1) and the second sensed data (DATA2).

4. The smart garment (100, 200, 300, 400) according to claim 2, further comprising an electrical component protection device (500) for accommodating the control module (120) and the calculation module (140, 1100).

5. The smart garment (100, 200, 300, 400) according to claim 1, further comprising:
a communication module (150), disposed at the smart garment body (110) to enable the smart garment (100, 200, 300, 400) to communicate with an external computer device (1000) so as to transmit the first sensed data (DATA1) and the second sensed data (DATA2) to the external computer device (1000).

6. The smart garment (100, 200, 300, 400) according to claim 5, wherein the sensing module (130) of the smart garment (100, 200, 300, 400) communicates with a calculation module (140, 1100) of the external computer device (1000) through the communication module (150), the calculation module (140, 1100) generates a calculation result (CR) according to the first sensed data (DATA1) and the second sensed data (DATA2), such that the control module (120) generates a control signal (CS) for controlling the smart garment body (110) according to the calculation result (CR).

7. The smart garment (100, 200, 300, 400) according to claim 6, wherein the calculation module (140, 1100) uses an artificial intelligence algorithm to generate the calculation result (CR) according to the first sensed data (DATA1) and the second sensed data (DATA2).

8. The smart garment (100, 200, 300, 400) according to claim 5, wherein the external computer device (1000) generates an operation instruction according to an input operation performed by a user, and the control module (120) generates a control signal (CS) according to the operation instruction for controlling the smart garment body (110).

9. The smart garment (100, 200, 300, 400) according to claim 1, wherein the first sensor (131) and the second sensor (132) are disposed at an inside surface and an outside surface with a chest position of the smart garment body (110).

10. The smart garment (100, 200, 300, 400) according to claim 1, wherein the smart garment body (110) comprises at least one signaling yarn (420, 600, 700), the signaling yarn (420, 600, 700) comprising:
a first staple fiber (610), having a stretching resistance of 26 to 40 strands and functioning as a first supporting material;
a first sheet conductor (620), enlacing a surrounding surface of the first staple fiber (610) in a spiral extending manner, and an aspect ratio of a cross section of the first sheet conductor (620) corresponding to the spiral extending manner is about 10 to 30; and
an insulating layer (730), surrounding the surrounding surface of the first staple fiber (610) for covering the first sheet conductor (620) and the first staple fiber (610).

11. The smart garment (100, 200, 300, 400) according to claim 10, wherein a material of the fist staple fiber (610) is one selected from polyester, polyamides, polyacrylonitriles, polyethylenes, polypropylenes, celluloses, proteins, elastic fibers, poly perfluoroethylene, polyparaphenylene benzoxazole, polyether ketone, carbon and glass fiber.

12. The smart garment (100, 200, 300, 400) according to claim 10, wherein a material of the first sheet conductor (620) is one selected from copper-nickel alloy, copper-tin alloy, copper-nickel-silicon alloy, copper-nickel-zinc alloy, copper-nickel-tin alloy, copper-chromium alloy, copper-silver alloy, nickel-brass alloy, phosphor bronze alloy, beryllium copper alloy, nickel-chromium alloy, copper-tungsten alloy and stainless steel.

13. The smart garment (100, 200, 300, 400) according to claim 10, wherein a material of the insulating layer (730) is one selected from polytetrafluoroethylene (PTFE, also known as Teflon®), ethylene tetrafluoroethylene (ETFE), polyethylene terephthalate (PET), polyvinyl chloride (PVC) and polyethylene (PE).

14. The smart garment (100, 200, 300, 400) according to claim 10, wherein a first conductive wire (620') is rolled for providing the first sheet conductor (620), wherein if a diameter of a circular section of the first conductive wire is denoted by X, a length of the cross section of the first sheet conductor (620) equals about 4X, and a width of the cross section of the first sheet conductor (620) equals about X/5.

15. The smart garment (100, 200, 300, 400) according to claim 1,wherein the smart garment body (110) comprising:
a touch control textile (410), disposed at the smart garment body (110) and electrically connected to the control module (120), the touch control textile (410) comprising:
a plurality of yarns (430) and a plurality of signaling yarns (420, 600, 700), wherein the touch control textile (410) worn by the yarns (430) and the signaling yarns (420, 600, 700), the signaling yarns (420, 600, 700) comprising:
a second staple fiber (610), having a stretching resistance of 26 to 40 strands and functioning as a second supporting material;
a second sheet conductor (620), enlacing a surrounding surface of the second staple fiber (610) in a spiral extending manner, wherein an aspect ratio of a cross section of the second sheet conductor (620) corresponding to the spiral extending manner is about 10 to 30.

16. The smart garment (100, 200, 300, 400) according to claim 15, wherein a material of the second staple fiber (610) is one selected from polyester, polyamides, polyacrylonitriles, polyethylenes, polypropylenes, celluloses, proteins, elastic fibers, poly perfluoroethylene, polyparaphenylene benzoxazole, polyether ketone, carbon and glass fiber.

17. The smart garment (100, 200, 300, 400) according to claim 15, wherein a material of the second sheet conductor (620) is one selected from copper-nickel alloy, copper-tin alloy, copper-nickel-silicon alloy, copper-nickel-zinc alloy, copper-nickel-tin alloy, copper-chromium alloy, copper-silver alloy, nickel-brass alloy, phosphor bronze alloy, beryllium copper alloy, nickel-chromium alloy, copper-tungsten alloy and stainless steel.

18. The smart garment (100, 200, 300, 400) according to claim 15, wherein a second conductive wire (620') is rolled for providing the second sheet conductor (620), wherein if a diameter of a circular section of the second conductive wire (620') is denoted by X, a length of the cross section of the second sheet conductor (620) equals about 4X, and a width of the cross section of the second sheet conductor (620) equals about X/5.

19. The smart garment (100, 200, 300, 400) according to claim 15, wherein a material of the second sheet conductor (620) is one selected from copper-nickel alloy, copper-tin alloy, copper-nickel-silicon alloy, copper-nickel-zinc alloy, copper-nickel-tin alloy, copper-chromium alloy, copper-silver alloy, nickel-brass alloy, phosphor bronze alloy, beryllium copper alloy, nickel-chromium alloy, copper-tungsten alloy and stainless steel.

20. The smart garment (100, 200, 300, 400) according to claim 1, further comprising an electrical component protection device (500) for accommodating the control module (120).
